# EUROPEAN PATENT SPECIFICATION

(11) **EP 1 919 910 B1**
(45) Date of publication and mention of the grant of the patent: **23.02.2011**
(21) Application number: 06762868.5
(22) Date of filing: 28.07.2006
(51) Int. Cl.: C07D 471/04, A61K 31/4353, A61P 35/00

(54) **AZABENZIMIDAZOLE DERIVATIVES, THEIR MANUFACTURE AND USE AS ANTI-CANCER AGENTS**
AZABENZIMIDALZOLDERIVATE, IHRE HERSTELLUNG UND IHRE VERWENDUNG ALS ANTIKREBSMITTEL
DERIVES DE L'AZABENZIMIDAZOLE, LEUR FABRICATION ET LEUR UTILISATION EN TANT QU'AGENTS ANTICANCEREUX

(30) Priority: 29.07.2005 EP 05016607
(43) Date of publication of application: 14.05.2008
(73) Proprietor: F. Hoffmann-La Roche AG, 4070 Basel (CH)
(72) Inventor: ENGH, Richard, NO-9012 Tromsoe (NO); HERTENBERGER, Hubert, 82362 Weilheim (DE); HONOLD, Konrad, 82377 Penzberg (DE); MASJOST, Birgit, CH-4052 Basel (CH); RUEGER, Petra, 82377 Penzberg (DE); SCHAEFER, Wolfgang, 68199 Mannheim (DE); SCHEIBLICH, Stefan, 82377 Penzberg (DE); SCHWAIGER, Manfred, 85368 Wang-Bergen (DE)
(74) Representative: Schreiner, Siegfried
(86) International application number: PCT/EP2006/007478
(87) International publication number: WO 2007/017143

(56) References cited:
- WO-A-03/035065

## Description

This invention relates to azabenzimidazole derivatives that inhibit the activity of protein kinases. Protein kinases are enzymes that catalyze the transfer of a phosphate group from ATP to an amino acid residue, such as tyrosine, serine, threonine, or histidine on a protein. Regulation of these protein kinases is essential for the control of a wide variety of cellular events including proliferation and migration.

### Background of the Invention

Inappropriate activation of tyrosine kinases is known to be involved in a variety of disease states including inflammatory, immunological, CNS disorders, or oncological disorders, or bone diseases. See for example Susva, M., et al., Trends Pharmacol. Sci. 21 (2000) 489-495; Biscardi, J.S., et al., Adv. Cancer Res. 76 (1999) 61-119.

The tyrosine kinases are a class of protein kinases. The Src family which consists of at least eight members (Src, Fyn, Lyn, Yes, Lck, Fgr, Hck and Blk) that participate in a variety of signaling pathways represents the major family of cytoplasmic protein tyrosine kinases (Schwartzberg, P.L., Oncogene 17 (1998) 1463-1468). The prototypical member of this tyrosine kinase family is Src, which is involved in proliferation and migration responses in many cell types (Sawyer, T., et al., Expert Opin. Investig. Drugs 10 (2001) 1327-1344). Src activity has been shown to be elevated in different cancers, e.g. breast, colon (>90%), pancreatic (>90%) and liver (>90%) tumors. Highly increased Src activity is also associated with metastasis (>90%) and poor prognosis. Antisense Src message impedes growth of colon tumor cells in nude mice (Staley, C.A., Cell Growth Differ. 8 (1997) 269-274), suggesting that Src inhibitors could slow tumor growth. Furthermore, in addition to its role in cell proliferation, Src also acts in stress response pathways, including the hypoxia response. Nude mice studies with colon tumor cells expressing antisense Src message have reduced vascularization (Ellis, L.M., et al., J. Biol. Chem. 273 (1998) 1052-1057), which suggests that Src inhibitors could be anti-angiogenic as well as anti-proliferative.

Src disrupts E-cadherin associated cell-cell interactions (Avizienyte, E., et al., Nat. Cell Biol. 4 (2002) 632-638). A low molecular weight Src inhibitor prevents this disruption thereby reducing cancer cell metastasis (Nam, J.S., et al., Clin. Cancer Res. 8 (2002) 2430-2436).

Src inhibitors may prevent the secondary injury that results from a VEGF-mediated increase in vascular permeability such as that seen following stroke (Eliceiri, B.P., et al., Mol. Cell. 4 (1999) 915-924; Paul, R., et al., Nat. Med. 7 (2001) 222-227).

Blockade of Src prevents dissociation of the complex involving Flk, VE- cadherin, and β-catenin with the same kinetics with which it prevents VEGF-mediated VP/edema and account for the Src requirement in VEGF- mediated permeability and provide a basis for Src inhibition as a therapeutic option for patients with acute myocardial infarction (Weis, S., et al., J. Clin. Invest. 113 (2004) 885-894).

Src also plays a role in osteoporosis. Mice genetically engineered to be deficient in Src production were found to exhibit osteopetrosis, the failure to resorb bone (Soriano, P., et al., Cell 64 (1991) 693-702; Boyce, B.F., et al., J. Clin. Invest. 90 (1992) 1622-1627). This defect was characterized by a lack of osteoclast activity. Since osteoclasts normally express high levels of Src, inhibition of Src kinase activity maybe useful in the treatment of osteoporosis (Missbach, M., et al., Bone 24 (1999) 437-449).

Low molecular weight inhibitors for protein kinases are widely known in the state of the art. For src inhibition such inhibitors are based on i.e. thieno-pyridine derivatives (US 2004/0242883); pyrido-pyrimidine derivatives (WO 04/085436); pyrido-pyrimidone derivatives (WO 04/041823); pyrimidine derivatives (WO 03/004492 and WO 01/00213); Quinazoline derivatives (WO 01/94341 and WO 02/016352); isoxazole derivatives (WO 02/083668) and pyrazole derivatives (WO 02/092573).

Some phenyl-aza-benzimidazoles are known as inhibitors of IgE-mediated immune response and suppressors of cytokines and leukocytes with antiproliferative effect from WO 04/024897. And some benzimidazole-pyrazoles and -indazoles are known as kinase inhibitors from WO 03/035065, especially as inhibitors against Kdr, Syk and Itk tyrosine kinases.

### Summary of the Invention

The present invention relates to azabenzimidazole derivatives of the general formula 1 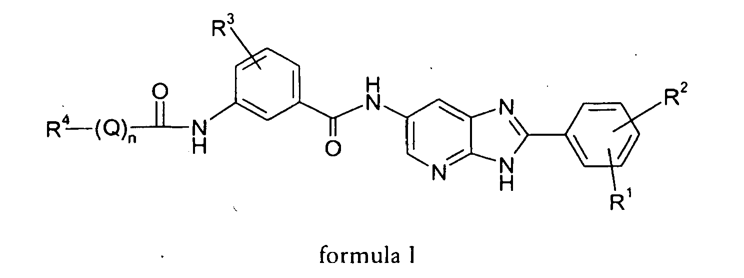 wherein,
- R¹ and R²: independently represent hydrogen, halogen, alkylamino, dialkylamino, alkoxy or alkyl, wherein the alkyl or the alkoxy are optionally substituted one or two times by alkoxy;
- R³: is hydrogen, halogen, alkoxy or alkyl, wherein the alkyl or the alkoxy are optionally substituted one or several times by halogen;
- Q: is alkylene or alkenylene;
- n: is 0 or 1;
- R⁴: is phenyl or pyridyl, said phenyl being optionally substituted once by phenyl, and all aromatic groups being optionally substituted one or several times by -CN, -CHO, -OH, -O-alkyl, -NO₂, -NH₂, -NH-alkyl, -alkylene-NH₂, alkyl, halogen or heterocyclyl;
and all pharmaceutically acceptable salts thereof.

The compounds according to this invention show activity as protein kinase inhibitors, e.g. as inhibitors of Src, Abl, EGFR, Raf or KDR kinases and in particular as inhibitors of Src family tyrosine kinases, and may therefore be useful for the treatment of diseases mediated by said tyrosine kinases, especially in the treatment of cancer.

Src family tyrosine kinases are known to be involved in a variety of disease states. Compounds of the present invention may be used as active agents in the prevention and therapy of, for example, transplant rejection, inflammatory bowel syndrome, rheumatoid arthritis, psoriasis, restenosis, allergic asthma, Alzheimer's disease, Parkinson, stroke, osteoporosis, benign hyperplasias and cancer including colon, breast, lung, prostate and pancreatic cancer and leukemia.

Objects of the present invention are the compounds of formula I and pharmaceutically acceptable salts and their enantiomeric forms, the preparation of the above-mentioned compounds, pharmaceutical compositions or medicaments containing them and their manufacture as well as the use of the above-mentioned compounds in the control or prevention of illnesses, especially of illnesses and disorders as mentioned above or in the manufacture of corresponding pharmaceutical compositions.

### Detailed Description of the Invention

As used herein, the term "alkyl" means a saturated, straight-chain or branched-chain hydrocarbon containing from 1 to 6 carbon atoms, preferably from1 to 4 carbon atoms, and more preferably 1 or 2 carbon atoms, such as methyl, ethyl, n-propyl, isopropyl, n-butyl, 2-butyl, t-butyl.

As used herein, the term "alkoxy" means an alkyl group as defined above which is connected via an oxygen atom. Examples are e.g. methoxy, ethoxy, isopropoxy and the like.

If said alkyl or alkoxy group is substituted one or two times by alkoxy it is substituted preferably by one alkoxy. Examples are e.g methoxy-methyl, ethoxymethyl, 2-methoxy-ethyl, 2-ethoxy-ethyl, 4-methoxy-butyl, 2-methoxy-butyl, 2-ethoxy-propyl, 3-propoxy-butyl, 2,3-dimethoxy-propyl, 2-ethoxy-3-methoxypropyl, 2,3-diethoxy-butyl, 1,2,3-trimethoxy-propyl, 2-methoxy-pentyl and the like.

If said alkyl or alkoxy group is substituted one or several times by halogen, it is substituted one to five, preferably one to three times by chlorine or fluorine, preferably by fluorine. Examples are difluoromethyl, trifluoromethyl, 2,2,2-trifluoroethyl, perfluorethyl, 2,2,2-trichloroethyl, 2-chloro-ethyl, 3-chloro-propyland the like, preferably difluoromethyl, trifluoromethyl, 2.,2,2-trifluoroethyl or perfluorethyl.

The term "alkylene" as used herein means a saturated, straight-chain or branched-chain, preferably straight-chain hydrocarbon containing from 1 to 5, preferably from 1 to 3, carbon atoms, such as methylene, ethylene, trimethylene; tetramethylene, pentamethylene, methylmethylene, 1-methyl-ethylene, 2-methyl-ethylene, 1-ethyl-ethylene, 2-ethyl-ethylene, 1-propyl-ethylene, 2-propyl-ethylene, 1-methyl-trimethylene, . 2-methyl-trimethylene, 1-ethyl-trimethylene, 2-ethyl-trimethylene, especially methylene, ethylene or trimethylene.

The term "alkenylene" as used herein means an unsaturated, straight-chain or branched-chain, preferably straight-chain hydrocarbon containing from 2 to 6, preferably from 2 to 3, carbon atoms. Examples of such "alkenylenes" are vinylene (ethenylene), allylene, isopropenylene, 1-propenylene, 2-methyl-1-propenylene, 1-butenylene, 2-butenylene, 3-butenylene, 2-ethyl-1-butenylene, 3-methyl-2-butenylene, 1-pentenylene, 2-pentenylene, 3-pentenylene, 4-pentenylene, 4-methyl-3-pentenylene, 1-hexenylene, 2-hexenylene, 3-hexenylene, 4-hexenylene, 5-hexenylene and the like, preferably vinylene (ethenylene), allylene, isopropenylene, 1-propenylene and 2-methyl-1-propenylene and especially vinylene (ethenylene).

As used herein, the term "halogen" means fluorine, chlorine, bromine and iodine, preferably fluorine, chlorine or bromine and more preferably fluorine and chlorine and still more preferably chlorine.

As used herein, the term "alkylamino" means an alkyl-NH- group wherein the alkyl is defined as above. Examples are e.g. methylamino, ethylamino, isopropylamino, butylamino and the like.

As used herein, the term "dialkylamino" means an (alkyl)₂N- group wherein the alkyl is defined as above. Examples are e.g. dimethylainino, ethyl-methyl-amino, diethylamino, methyl-isopropyl-amino and the like.

As used herein, the term "a therapeutically effective amount" of a compound means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 Kg, a daily dosage of about 10 mg to about 10,000 mg, preferably from about 200 mg to about 1,000 mg, should be appropriate, although the upper limit may be exceeded when indicated. The daily dosage can be administered as a single dose or in divided doses, or for parenteral administration, it may be given as continuous infusion.

As used herein, a "pharmaceutically acceptable carrier" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention are contemplated. Supplementary active compounds can also be incorporated into the compositions.

Preferably the postion of R³ in formula I is para to the R⁴-(Q)ₙ-C(O)NH-substituent.

An embodiment of the invention are compounds according to formula I, wherein
- R²: is hydrogen; and
- R³: is halogen or alkyl.

An embodiment of the invention are compounds according to formula I, wherein
- R²: is hydrogen;
- R³: is halogen, preferably chlorine, or alkyl; and
- Q: is alkenylene.

Another embodiment of the invention are compounds according to formula I, wherein
- R¹ and R²: are hydrogen;
- R³: is halogen or alkyl; and
- Q: is alkenylene.

Another embodiment of the invention are compounds according to formula I, wherein
- R³: is alkyl.

Another embodiment of the invention are compounds according to formula I, wherein
- R²: is hydrogen; and
- R³: is alkyl.

Another embodiment of the invention are compounds according to formula I, wherein
- R¹ and R²: are hydrogen;
- R³: is alkyl; and
- Q: is alkenylene.

Another embodiment of the invention are compounds according to formula I, wherein
- R³: is chlorine.

An embodiment of the invention are compounds according to formula I, wherein
- R²: is hydrogen; and
- R³: is chlorine.

Another embodiment of the invention are compounds according to formula I, wherein
- R¹ and R²: are hydrogen;
- R³: is chlorine; and
- Q: is alkenylene.

Another embodiment of the invention are compounds according to formula I, wherein
- n: is 1.

Another embodiment of the invention are compounds according to formula I, wherein
- R²: is hydrogen;
- R³: is halogen or alkyl; and
- n: is 1.

Another embodiment of the invention are compounds according to formula I, wherein
- R¹ and R²: are hydrogen;
- R³: is halogen or alkyl;
- Q: is alkenylene; and
- n: is 1.

Another embodiment of the invention are compounds according to formula I, wherein
- R³: is alkyl; and
- n: is 1.

Another embodiment of the invention are compounds according to formula I, wherein
- R²: is hydrogen;
- R³: is alkyl; and
- n: is 1.

Another embodiment of the invention are compounds according to formula I, wherein
- R¹ and R²: are hydrogen;
- R³: is alkyl;
- Q: is alkenylene; and
- n: is 1.

Another embodiment of the invention are compounds according to formula I, wherein
- R¹ and R²: are hydrogen;
- R³: is alkyl;
- R⁴: is phenyl, said phenyl being optionally substituted once by -NO₂;
- Q: is alkenylene; and
- n: is 1.

Another embodiment of the invention are compounds according to formula I, wherein
- R¹ and R²: are hydrogen;
- R³: is alkyl;
- R⁴: is pyridyl;
- Q: is alkenylene; and
- n: is 1.

Another embodiment of the invention are compounds according to formula I, wherein
- R³: is chlorine; and
- n: is 1.

Another embodiment of the invention are compounds according to formula I, wherein
- R²: is hydrogen;
- R³: is chlorine; and
- n: is 1.

Another embodiment of the invention are compounds according to formula I, wherein
- R¹ and R²: are hydrogen;
- R³: is chlorine;
- Q: is alkenylene; and
- n: is 1.

Another embodiment of the invention are compounds according to formula I, wherein
- R¹ and R²: are hydrogen;
- R ³: is chlorine;
- R⁴: is phenyl, said phenyl being optionally substituted once by -NO₂;
- Q: is alkenylene; and
- n: is 1.

Another embodiment of the invention are compounds according to formula I, wherein
- R¹ and R²: are hydrogen;
- R³: is chlorine;
- R⁴: is pyridyl;
- Q: is alkenylene; and
- n: is 1.

Another embodiment of the invention are compounds according to formula I, wherein
- R¹ and R²: are hydrogen;
- R³: is chlorine or alkyl;
- R⁴: is phenyl, said phenyl being optionally substituted once by -NO₂;
- Q: is alkenylene; and
- n: is 1.

Such compounds, for example, may be selected from the group consisting of:
2-Chloro-5-[(E)-(3-phenyl-acryloyl)amino]-*N-*(2-phenyl-3*H-*imidazo[4,5-*b*]pyridin-6-yl)-benzamide;
2-Methyl-5-[(E)-(3-phenyl-acryloyl)amino]-*N-*(2-phenyl-3*H-*imidazo[4,5-*b*] pyridin-6-yl)-benzamide;
2-Methyl-5-[(E)-3-(3-nitro-phenyl)-acryloylamino]-N-(2-phenyl-3H-imidazo[4,5-b]pyridin-6-yl)-benzamide;
2-Methyl-5-[(E)-3-(2-nitro-phenyl)-acryloylamino]-N-(2-phenyl-3H-imidazo[4,5-b]pyridin-6-yl)-benzamide; and
2-Methyl-5-[(E)-3-(4-nitro-phenyl)-acryloylamino]-N-(2-phenyl-3H-imidazo[4,5-b] pyridin-6-yl)-benzamide.

Another embodiment of the invention are compounds according to formula I, wherein
- R¹ and R²: are hydrogen;
- R³: is chlorine or alkyl;
- R⁴: is pyridyl,
- Q: is alkenylene; and
- n: is 1.

Such compounds, for example, may be selected from the group consisting of:
2-Methyl-*N*-(2-phenyl-3*H*-imidazo[4,5-*b*]pyridin-6-yl)-5-((E)-3-pyridin-4-yl-acryloylamino)-benzamide; and
2-Methyl-*N*-(2-phenyl-3*H*-imidazo[4,5*-b*]pyridin-6-yl)-5-((E)-3-pyridin-3-yl-acryloylamino)-benzamide

Another embodiment of the invention are compounds according to formula I, wherein
- n: is 0.

Another embodiment of the invention are compounds according to formula I, wherein
- R²: is hydrogen;
- R³: is halogen or alkyl; and
- n: is 0.

Another embodiment of the invention are compounds according to formula I, wherein
- R¹ and R²: are hydrogen;
- R³: is halogen or alkyl; and
- n: is 0.

Another embodiment of the invention are compounds according to formula I, wherein
- R³: is alkyl; and
- n: is 0.

Another embodiment of the invention are compounds according to formula I, wherein
- R²: is hydrogen;
- R³: is alkyl; and
- n: is 0.

Another embodiment of the invention are compounds according to formula I, wherein
- R¹ and R²: are hydrogen;
- R³: is alkyl; and
- n: is 0.

Another embodiment of the invention are compounds according to formula I, wherein
- R¹ and R²: are hydrogen;
- R³: is alkyl;
- R⁴: is phenyl, said phenyl being optionally substituted once by heterocyclyl; and
- n: is 0.

Another embodiment of the invention are compounds according to formula I, wherein
- R¹ and R²: are hydrogen;
- R³: is alkyl;
- R⁴: is phenyl, said phenyl being substituted once by phenyl; and
- n: is 0.

Another embodiment of the invention are compounds according to formula I, wherein
- R³: is chlorine; and
- n: is 0.

Another embodiment of the invention are compounds according to formula I, wherein
- R²: is hydrogen;
- R³: is chlorine; and
- n: is 0.

Another embodiment of the invention are compounds according to formula I, wherein
- R¹ and R²: are hydrogen;
- R³: is chlorine; and
- n: is 0.

Another embodiment of the invention are compounds according to formula I, wherein
- R¹ and R²: are hydrogen;
- R³: is chlorine;
- R⁴: is phenyl, said phenyl being optionally substituted once by heterocyclyl; and
- n: is 0.

Another embodiment of the invention are compounds according to formula I, wherein
- R¹ and R²: are hydrogen;
- R³: is chlorine;
- R⁴: is phenyl, said phenyl being substituted once by phenyl; and
- n: is 0.

Another embodiment of the invention are compounds according to formula I, wherein
- R¹ and R²: are hydrogen;
- R³: is chlorine or alkyl;
- R⁴: is phenyl, said phenyl being optionally substituted once by heterocyclyl; and
- n: is 0.

Such compounds, for example, may be selected from the group consisting of:
2-Methyl-5-(4-morpholin-4-yl-benzoylamino)-*N-*(2-phenyl-3*H-*imidazo[4,5-*b*] pyridin-6-yl)-benzamide;
5-Benzoylamino-2-methyl-*N*-(2-phenyl-3*H*-imidazo[4,5-*b*]pyridin-6-yl)-benzamide;
2-Chloro-5-(4-morpholin-4-yl-benzoylamino)-*N-*(2-phenyl-3*H-*imidazo[4,5-*b*]pyridin-6-yl)-benzamide; and
5-Benzoylamino-2-chloro-*N-*(2-phenyl-3*H-*imidazo[4,5*-b*]pyridin-6-yl)-benzamide.

Another embodiment of the invention are compounds according to formula I, wherein
- R¹ and R²: are hydrogen;
- R³: is chlorine or alkyl;
- R⁴: is phenyl, said phenyl being substituted once by phenyl; and
- n: is 0.

Such compounds, for example, may be selected from the group consisting of:
Biphenyl-3-carboxylic acid [4-methyl-3-(2-phenyl-3*H*-imidazo[4,5-*b*]pyridin-6-ylcarbamoyl)-phenyl]-amide;
Biphenyl-4-carboxylic acid [4-methyl-3-(2-phenyl-3*H-*imidazo[4,5*-b*]pyridin-6-ylcarbamoyl)-phenyl]-amide;
Biphenyl-4-carboxylic acid [4-chloro-3-(2-phenyl-3*H-*imidazo[4,5*-b*]pyridin-6-ylcarbamoyl)-phenyl]-amide; and
Biphenyl-3-carboxylic acid [4-chloro-3-(2-phenyl-3*H-*imidazo[4,5*-b*]pyridin-6-ylcarbamoyl)-phenylJ-amide.

Another embodiment of the invention are compounds according to formula I, wherein
- R¹ and R²: are hydrogen;
- R³: is halogen, preferably chlorine, or alkyl, preferably methyl;
- R⁴: is pyridyl or phenyl, said phenyl being optionally substituted once by phenyl, heterocyclyl or -NO₂; and
- Q: is alkenylene.

Another embodiment of the invention are compounds according to formula I, wherein
- n: is 1; and
- R⁴: is pyridyl.

Another embodiment of the invention are compounds according to formula I, wherein
- R¹ and R²: are hydrogen;
- R³: is halogen or alkyl;
- R⁴: is pyridyl;
- Q: is alkenylene; and
- n: is 1.

Another embodiment of the invention are compounds according to formula I, wherein
- R⁴: is phenyl, said phenyl being optionally substituted once by phenyl or heterocyclyl; and
- n: is 0.

Another embodiment of the invention are compounds according to formula I, wherein
- R¹ and R²: are hydrogen;
- R³: is halogen or alkyl;
- R⁴: is phenyl, said phenyl being optionally substituted once by phenyl or heterocyclyl; and
- Q: is alkenylene; and
- n: is 1.

Another embodiment of the invention is a process for the manufacture of the compounds of formula, wherein
(a) the compound of formula IX, 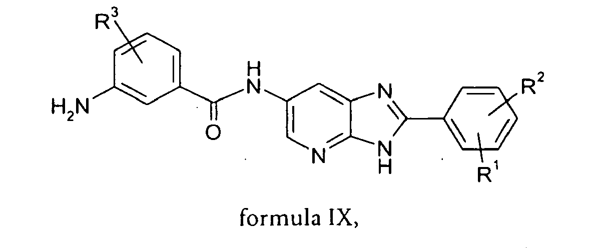 wherein R¹, R² and R³ have the significance as given above for formula I,
   is reacted with a compound of formula X, 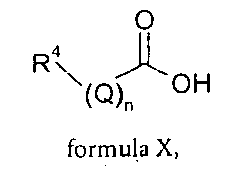 wherein R⁴, Q and n have the significance given above for formula I, and wherein the carboxylic acid group is activated before the reaction, to give the respective compound of formula I,
(b) said compound of formula I is isolated from the reaction mixture, and
(c) if desired, converted into a pharmaceutically acceptable salt.

The derivatives of the general formula I or a pharmaceutically acceptable salt thereof, may be prepared by any process known to be applicable for the preparation of chemically-related compounds by the one skilled in the art. Such processes, when used to prepare the derivatives of formula I, or a pharmaceutically-acceptable salt thereof, are provided as a further feature of the invention and are illustrated by the following representative examples of scheme 1, in which, unless otherwise stated R¹, R², R³, R⁴, Q and n have the significance given herein before for formula I. Necessary starting materials may be obtained by standard procedures of organic chemistry. The preparation of such starting materials is described within the accompanying examples. Alternatively necessary starting materials are obtainable by analogous procedures to those illustrated which are within the ordinary skill of an organic chemist.

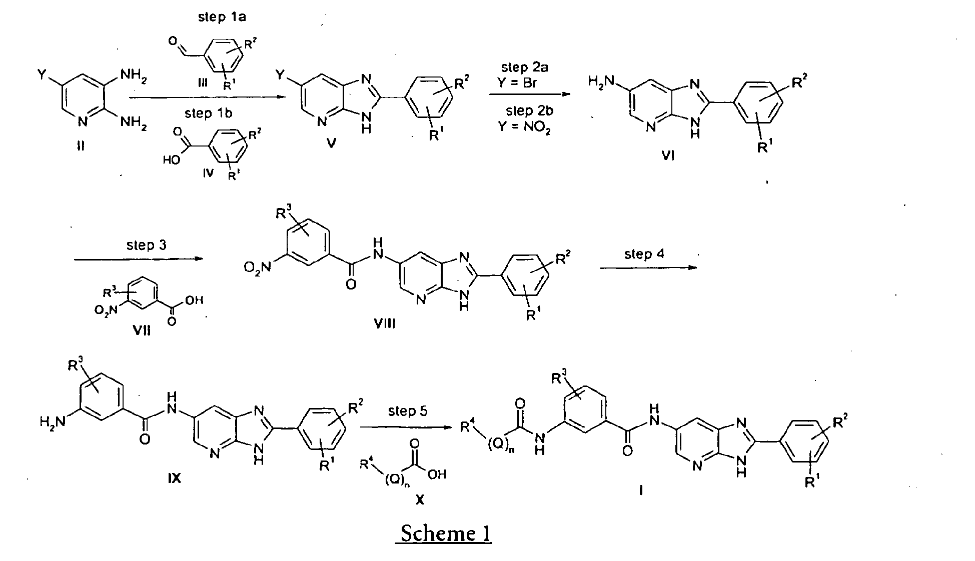

In scheme 1, R¹, R², R³, R⁴, Q and n have the significance as given above for formula I and Y is bromine (for the route via step 2a) or nitro (for the route via step 2b).

**Step 1a:** Condensation of an aromatic aldehyde of formula III with a 2,3-diamino-pyridine derivative of formula II can carried out at elevated temperatures from 60 to 200°C in a suitable solvent like acetonitrile, nitrobenzene, dimethylformamide (DMF), dimethylsulfoxide (DMSO), xylene, or methoxyethanol, optionally in the presence of an oxidizing agent like oxygen or an iron (III) salt or sulfur, or 2,3-dichloro-5,6-dicyano-1,4-benzoquinone (DDQ) to give the compounds of formula V.

**Step 1b:** The condensation with an aromatic carboxylic acid of formula IV, or a suitable derivative thereof, with a 2,3-diamino-pyridine derivative of formula II can be achieved at temperatures in the range of 100-220°C with a condensation reagent like polyphosphoric acid, POCl₃, or P₄O₁₀, optionally in mixture with methane sulfonic acid to give the compounds of formula V.

**Step 2a:** In the compounds of formula V, wherein Y is bromine, such bromine can be replaced by an amino group by heating in aqueous ammonia in the presence of a catalyst like CuSO₄ or CuI to give the compounds of formula VI. A solubilizing cosolvent like N-methyl-pyrrolidone (NMP) or N,N-dimethyl acetamide can be added, and the reaction is carried out at temperatures of 100-180°C in a closed vessel.

Alternatively, the amino functionality may be introduced in protected form as a tert.-butoxycarbonylamino substituent via coupling under standard Hartwig/Buchwald conditions (for example, with a base like sodium tert. butoxide and a palladium catalyst like Pd₂(dba)₃ and a phosphine ligand like tri-tert. butyl phosphane).

**Step 2b and step 4**: For the compounds of formula V wherein Y is nitro (Step 2b), and the compounds of formula VIII (Step 4), the reduction of the nitro group is accomplished by standard conditions such as heterogeneous hydrogenation with Pd on charcoal as the catalyst, in solvents like methanol, ethanol, tetrahydrofuran (THF), or ethyl acetate, at room temperature or up to 80°C; or by homogeneous hydrogenation with a Pd catalyst and triethyl ammonium formate in a solvent like methanol at reflex conditions. The reduction can also be carried out with base metals like iron or tin in acidic media like acetic acid or aqueous HCl, from room temperature to 120°C. Another suitable reductant would be ammonium sulfide in water or methanol, or tin (II) chloride in dimethylformamide (DMF) or in aqueous HCl. This reduction reaction yields the corresponding the compounds of formula VI (Step 2b), or the compounds of formula IX (Step 4),

**Step 3 and step 5:** Acylation of the amino moiety on the compounds of formula VI (Step 3), and the compounds of formula IX (Step 5), can be done with the appropriate carboxylic acids of formula VII (Step 3), or the acids of formula X (Step 5), in a two step procedure. In the first step, the carboxylic acid becomes activated. This reaction is carried out in an inert solvent or diluent, for example, in dichloromethane, dioxane, tetrahydrofuran (THF), N,N-dimethylformamide (DMF), or N-methylpyrrolidone (NMP), in the presence of an activating agent. Suitable activating agents are, for example, oxalyl or thionyl chloride, isobutyl chloroformate, N-hydroxybenzotriazole, N,N'-carbonyldiimidazole, dicyclohexylcarbodiimide, N-(3-dimethylaminopropyl)-N'-ethylcarbodiimide (EDC), 2-morpholino-ethyl-isocyanide (MEI) and the like. Other activating agents can also be used and are well known to the skilled artist. The activated carboxylic acid derivative (e.g. the acid chloride) can be sometimes isolated as intermediate or they are sometimes commercially available. Nevertheless the reaction is often carried out in a one-pot procedure without isolation of the activated carboxylic acid intermediate. In the second step, the amine of formula VI (Step 3), or the amine of formula IX (Step 5), is reacted with the appropriate activated carboxylic acid (VI in Step 3 or X in Step 5) yielding the compounds of formula VIII (Step 3) or the compounds of formula I (Step 5). This reaction can also be carried out in pyridine, optionally in the presence of a base like triethyl amine or ethyl diisopropyl amine, and can be catalyzed sometimes by N,N-dimethylaminopyridine (DMAP) and the like.

If an excess of activated carboxylic acid derivative is used, simultaneous acylation on the heterocyclic core can occur, e.g. on N-1 or N-3. Such a bis-acylated intermediate can be cleaved easily to the desired mono-acylated compound by subsequent treatment with ammonia in water or methanol at room temperature.

Certain substituents on the group R¹, R², R³ and R⁴ may not be inert to the conditions of the synthesis sequences described above and may require protection by standard protecting groups known in the art. For instance, an amino or hydroxyl group maybe protected as a tert.-butoxycarbonyl derivative. Alternatively, some substituents may be derived from others at the end of the reaction sequence. For instance, a compound of formula I may be synthesized bearing a nitro- substituent, which substituent is finally converted to an amino by standard procedures.

The compounds of the general formula I can contain one or several chiral centers and can then be present in a racemic or in an optically active form. The racemates can be separated according to known methods into the enantiomers. For instance, diastereomeric salts which can be separated by crystallization are formed from the racemic mixtures by reaction with an optically active acid such as e.g. D- or L-tartaric acid, mandelic acid, malic acid, lactic acid or camphorsulfonic acid. Alternatively separation of the enantiomers can also be achieved by using chromatography on chiral HPLC-phases which are commercially available.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula I and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethylammonium hydroxide, especially from sodium. The chemical modification of a pharmaceutical compound into a salt is a technique well known to pharmaceutical chemists in order to obtain improved physical and chemical stability, hygroscopicity, flowability and solubility of compounds. It is for example described in Stahl, P. H., and Wermuth, G., (editors), Handbook of Pharmaceutical Salts, Verlag Helvetica Chimica Acta (VHCA), Zürich, (2002) or Bastin, R.J., et al., Organic Proc. Res. Dev. 4 (2000) 427-435.

The compounds according to this invention and their pharmaceutically acceptable salts can be used as medicaments, e.g. in the form of pharmaceutical compositions. The pharmaceutical compositions can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The above-mentioned pharmaceutical preparations can be obtained by processing the compounds according to this invention with pharmaceutically acceptable, inorganic or organic carriers. Lactose, corn starch or derivatives thereof, talc, stearic acids or its salts and the like can be used, for example, as such carriers for tablets, coated tablets, dragées and hard gelatine capsules. Suitable carriers for soft gelatine capsules are, for example, vegetable oils, waxes, fats, semi-solid and liquid polyols and the like. Depending on the nature of the active substance no carriers are, however, usually required in the case of soft gelatine capsules. Suitable carriers for the production of solutions and syrups are, for example, water, polyols, glycerol, vegetable oil and the like. Suitable carriers for suppositories are, for example, natural or hardened oils, waxes, fats, semi-liquid or liquid polyols and the like.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

An embodiment of the invention is a pharmaceutical composition containing one or more compounds according to formula I as active ingredients together with pharmaceutically acceptable carriers.

Another embodiment of the invention is said pharmaceutical composition for the treatment of diseases mediated by an inappropriate activation of src family tyrosine kinases.

Another embodiment of the invention is said pharmaceutical composition for the treatment of inflammatory-, immunological-, CNS disorders or bone diseases.

Another embodiment of the invention is said pharmaceutical composition for the treatment of cancer.

Another embodiment of the invention is said pharmaceutical composition for the treatment of colorectal cancer, breast cancer, lung cancer, prostate cancer, pancreatic cancer, gastric cancer, bladder cancer, ovarian cancer, melanoma, neuroblastoma, cervical cancer, kidney cancer or renal cancer, leukemias or lymphomas.

Another embodiment of the invention is the use of one or more compounds according to formula I for the manufacture of pharmaceutical compositions for the treatment of diseases mediated by an inappropriate activation of src family tyrosine kinases.

Another embodiment of the invention is the use of one or more compounds according to formula I for the manufacture of pharmaceutical compositions for the treatment of cancer.

Another embodiment of the invention is the use of one or more compounds according to formula I for the manufacture of pharmaceutical compositions for the treatment of colorectal cancer, breast cancer, lung cancer, prostate cancer, pancreatic cancer, gastric cancer, bladder cancer, ovarian cancer, melanoma, neuroblastoma, cervical cancer, kidney cancer or renal cancer, leukemias or lymphomas.

Another embodiment of the invention is the use of one or more compounds according to formula I for the manufacture of pharmaceutical compositions for the treatment of inflammatory-, immunological-, CNS disorders or bone diseases.

Another embodiment of the invention are compounds according to formula (I) for use in a method of treatment as src family tyrosine kinase inhibitors.

Another embodiment of the invention are compounds according to formula (I) for use in a method of treatment as cell signaling-regulating and anti-proliferating agents.

Another embodiment of the invention are compounds according to formula (I) for use in a method of treatment of inflammatory-, immunological-, CNS disorders or bone diseases.

Another embodiment of the invention are compounds according to formula (I) for use in a method of treatment of cancer.

Another embodiment of the invention is a pharmaceutical composition comprising a therapeutically effective amount of a compound according to formula I as active ingredients and a pharmaceutically acceptable carrier.

Another embodiment of the invention are compounds according to formula (I) for use in a method of treatment of colorectal cancer, breast cancer, lung cancer, prostate cancer, pancreatic cancer, gastric cancer, bladder cancer, ovarian cancer, melanoma, neuroblastoma, cervical cancer, kidney cancer or renal cancer, leukemias or lymphomas comprising administering to a person in need thereof a therapeutically effective amount of a compound according to formula I.

A pharmaceutical preparation was obtained e.g. by using the following procedure:
1. Weigh 4.0 g glass beads in custom made tube GL 25, 4 cm (the beads fill half of the tube).
2. Add 50 mg compound, disperse with spatulum and vortex.
3. Add 2 ml gelatin solution (weight beads: gelatin solution = 2:1) and vortex.
4. Cap and wrap in aluminium foil for light protection.
5. Prepare a counter balance for the mill.
6. Mill for 4 hours, 20/s in a Retsch mill (for some substances up to 24 hours at 30/s).
7. Extract suspension from beads with two layers of filter (100 µm) on a filter holder, coupled to a recipient vial by centrifugation at 400 g for 2 min.
8. Move extract to measuring cylinder.
9. Repeat washing with small volumes(here 1 ml steps) until final volume is reached or extract is clear.
10. Fill up to final volume with gelatin and homogenise.

The above described preparation yields micro-suspensions of the compounds of formula I with particle sizes between 1 and 10 µm. The suspensions are suitable for oral applications and were used in the in vivo pharmacokinetic testings described below.

### Pharmacological activity:

The activity of the compounds according to this invention as protein kinase inhibitors, in particular for the src-family tyrosine kinases, was shown by using the following assay.

### SRC-Inhibitor-Assay Parameters:

### Reaction mixture:

| | |
|---|---|
| ATP | 5 µM |
| Peptide (Ro + Ja133-Ro): | 10 µM |
| Ja133-Ro | 196 nM |
| Ro | 9.8 µM |
| PT66 | 230 ng/ml |
| Assay buffer: | 4 mM MgCl2 |
| | 2 mM TCEP |
| | 50 mM HEPES |
| | 0,1 % Tween 20 |
| | pH 7.3 |
| | |
| Enzyme: | 2.5 U/ml |
| | |
| Inhibitor: | max. 25 µM |
| | min. 0.42 nM |

### Material:

| | | |
|---|---|---|
| Eu-labelled phosphotyrosine antibody: | | - for Lck Cisbio Mab PT66-K, |
| | | - for Src EG&G Wallac PT66 Eu-W1024 |
| | | (all commercially available). |
| | | |
| Peptides: | Ro: | NH₂-A-E-E-E-I-Y-G-E-F-E-A-K-K-K-K-CONH₂, and |
| | Ja133-Ro: | Ja133-G-Aminocaprylic acid-A-E-E-E-1-Y-G-E-F-E-A-K-K-K-K-CONH₂, wherein Ja133 is LightCycler-Red 640-N-hydroxy succinimide ester; |

whereby both peptides were synthesized by an optimized solid phase peptide synthesis protocol (Merrifield, Fed. Proc. Fed. Amer. Soc. Exp. Biol. 21 (1962) 412) on a Zinsser SMP350 peptide synthesizer. Shortly, the peptide was assembled on 160 mg (22.8 µmol scale) of a Rink-Linker modified polystyrene solid phase by repeatedly conjugating an twenty fold excess of amino acids each protected by temporary piperidine labile Fmoc- and permanent acid labile tert-Bu-, BOC- and O-tert-Bu-groups depending on the side chain function. The substrate sequence AEEEIYGEFEAKKKK was N-terminal additionally mounted with the spacer amino acids Aminocaprylic acid and Glycin. After cleavage of the N-terminal temporary protecting group the still attached and protected peptide was labeled with a 1.5 fold amount of LightCycler-Red 640-N-hydroxy succinimide ester (purchased from Roche Diagnostics GmbH) and triethylamine. After 3 hrs. the resin was washed with Dimethylformamide and Isopropanol until the eluates of the blue resin got colourless. The fully protected and labeled peptide was removed from the solid phase and released from the permanent protecting groups by treatment with a mixture of 80% trifluoroacetic acid, 10% Ethanedithiol, 5% Thioanisol and 5% Water. The substrate was finally isolated by a preparative reverse phase HPLC purification. The purification yielded 12.2 mg RP-HPLC single peak pure blue material (lyophilisate). The identity was proven by MALDI mass spectroscopy [2720.0].

| | |
|---|---|
| Enzymes: | Upstate Lck (p56^{lck}, active), Upstate Src (p60^{c-src}, partially purified) were purchased from UB1, Upstate Biotech, Inc.. |

Time-resolved Fluorescence Assay: Reader: Perkin Elmer, Wallac Viktor 1420-040 multilabel counter; Liquid handling system: Beckman Coulter, Biomek 2000.

ATP, Tween^{™} 20, 4-(2-hydroxyethyl)-1-piperazineethanesulfonic acid (HEPES) were purchased from Roche Molecular Biochemicals, MgCl₂ and MnCl₂ were purchased from Merck Eurolab, Tris(2-carboxyethyl)phosphine hydrochloride (TCEP) was purchased from Pierce, 384 Well low volume fluorescence plates was purchased from Falcon.

### Assay Description:

At first the enzyme is pre-incubated for 15 min. at 15°C in aqueous solution with corresponding amounts of inhibitors according to this invention. Then the phosphorylation reaction is started by adding a reaction mixture, containing ATP, Peptide and PT66, and subsequent shaking. The proceeding of this reaction is immediately monitored using time resolved fluorescence spectroscopy in a suitable well plate reader.

The IC₅₀-values can be obtained from the reaction rates by using a non-linear curve fit (XLfit software (ID Business Solution Ltd., Guilford, Surrey, UK))

| **Example-No.** | **IC50 src** **[µM]** |
|---|---|
| 1-3 | 0.043 |
| 2-4 | 0.064 |
| 1-2, 1-4, 1-5, 1-6,2-1,2-3 | 0.01-0.10 |
| 1-1,2-3 | 0.10-1.00 |

| **Example-No.** | **IC50 lck** **[µM]** |
|---|---|
| 1-3 | 0.064 |
| 2-4 | 0.200 |
| 1-1, 1-2, 1-4, 1-5, 2-1, 2-2, 2-5 | 0.01-0.10 |
| 1-6 | 0.10-1.00 |

The following examples are provided to aid the understanding of the present invention, the true scope of which is set forth in the appended claims. It is understood that modifications can be made in the procedures set forth without departing from the spirit of the invention.

### Experimental Procedures:

### Starting materials

### Example a) 6-Nitro-2-phenyl-3H-imidazo[4,5-b]pyridine

14.05 g 2,3-diamino-5-nitropyridine and 9.68 g benzaldehyde in 250 ml nitrobenzene were heated to 140-150 °C for 15 hrs. The solvent is removed by vacuum distillation and the residue is dispersed in ethyl acetate, filtered, and the filter residue washed thoroughly with ethyl acetate.
Yield 16.0 g

### Example b) 2-phenyl-3H-imidazo[4,5-b]pyridin-6-ylamine

1.2.0 g 6-nitro-2-phenyl-3H-imidazo[4,5-b]pyridine were dissolved in 1 I acetic acid. 18 g iron powder were added and the mixture heated to 80 °C with stirring. After 2 hrs the mixture was cooled to room temperature and filtered over Celite. The celite pad was washed with methanol and the combined filtrates were evaporated. The residue was dissolved methanol / dichloromethane 1:1 and filtered over silica. The filtrate was concentrated to a volume of 100 ml, the resulting precipitate collected by filtration and washed with methanol.
Yield 7.68 g

### Example c) 2-Chloro-5-nitro-N-(2-phenyl-3H-imidazo[4,5-b]pyridin-6-yl)-benzamide

1,00 g 2-phenyl-3H-imidazo[4,5-b]pyridin-6-ylamine from Example b) (0.38 mmol,) in 15 ml dry pyridine were treated at room temperature with a solution of 2,30 g (2.2 equivalents) 2-chloro-5-nitrobenzoyl chloride in 5 ml pyridine. The mixture was stirred over night at room temperature. The solvent was evaporated, the residue was dissolved in 5 ml methanol and 2 ml conc. aqueous ammonia were added to cleave bis-acylated byproducts. After stirring for 2 hrs all solvents were evaporated and the residue again dissolved in methanol. Upon addition of water the product precipitated. It was isolated by filtration and thoroughly washed with water and subsequently with ether, and finally dried.
Yield 0,735 g.

### Example d) 5-Amino-2-chloro-N-(2-phenyl-3H-imidazo[4,5-b]pyridin-6-yl)-benzamide

700 mg of the product from Example c) in 30 ml ethanol were cooled in a water bath at room temperature. 1.20 g tin (II) chloride and 2 ml conc. HCl were slowly added and the mixture was stirred for 1 hr at 40 °C. The solvent was evaporated and the residue adjusted to pH 5 with aqueous sodium carbonate solution. After dilution with water the precipitate was isolated by filtration and washed with water and ether. The filter residue was dispersed in methanol and filtered again over a pad of Celite. The Celite pad was washed thoroughly with methanol, and the combined filtrates were evaporated. The residue was purified by preparative HPLC.
Yield 192 mg.

### Example e) 2-Methyl-5-nitro-N-(2-phenyl-3H-imi dazo[4,5-b]pyridin-6-yl)-benzamide

Analogous to Example c) from 1.74 g 2-phenyl-3H-imidazo[4,5-b]pyridin-6-ylamine and 3,31g 2-methyl-5-nitrobenzoyl chloride, which was prepared as follows:
To 3.00 g 2-methyl-5-nitrobenzoic acid in 50 ml dichloromethane were added one drop of DMF and then dropwise at room temperature 2.52 g oxalyl chloride. The mixture was stirred for 2 hrs and then evaporated to give a residue of 3.558 g crude 2-methyl-5-nitrobenzoyl chloride.
Yield 1.878 g of the title product

### Example f) 5-Amino-2-methyl-N-(2-phenyl-3H-imidazo[4,5-b]pyridin-6-yl)-benzamide

1.80 g of the product from Example e) were dissolved in 30 ml methanol and 30 ml THF and hydrogenated with 0.5 g 10% palladium on charcoal at room temperature for 45 min. The catalyst was removed by filtration over a small pad of silica and the silica was washed thoroughly with methanol / THF 1:1. Evaporation of the filtrates gave 1.11 g of the title product.

### Final products

### Example 1-1:

### 2-Methyl-N-(2-phenyl-3H-imidazo[4,5-b]pyridin-6-yl)-5-((E)-3-pyridin-4-yl-acryloylamino)-benzamide

To 200 mg (E)-3-Pyridin-4-yl-acrylic acid in 5 ml dichloromethane were added one drop of dimethylformamide (DMF) and then dropwise at room temperature 204 mg oxalyl chloride. The mixture was stirred for 2 hrs and then evaporated to give a residue of crude (E)-3-Pyridin-4-yl-acryloyl chloride. 54 mg of this acid chloride were given dropwise to a solution of 50 mg 5-Amino-2-methyl-N-(2-phenyl-3H-imidazo[4,5-b]pyridin-6-yl)-benzamide from Example f) in 2 ml dry pyridine at room temperature. After stirring over night the mixture was evaporated to dryness, dissolved in 3 ml methanol and stirred with 1 ml conc. aqueous ammonia at room temperature to cleave bis-acylated byproducts. After one hr all solvents were removed under vacuum and the residue dispersed in water. The crude product was isolated by filtration and washed thoroughly with water and subsequently with ether to give, after drying, 49.9 mg of the title product.

Using the experimental conditions reported above (Example 1-1) and the appropriate starting materials, the following derivatives were prepared:

| **Example No.** | **Systematic name** | **¹H-NMR** |
|---|---|---|
| 1-1 | 2-Methyl-*N-*(2-phenyl-3*H*-imidazo[4,5-*b*]pyridin-6-yl)-5-((E)-3-pyridin-4-yl-acryloylamino)-benzamide | (400 MHz, D6-DMSO) 13.55 (br s) and 13.09 (br s, together 1H), 10.66 (br s) and 10.56 (br s, together 1H), 10.49 (s, 1H), 8.86 - 8.39 (m, 4H), 8.21 (m, 2H), 7.88 (s, 1H), 7.75 (d, 1H), 7.58 (m, 6H), 7.32 (d, 1H), 7.05 (d, 1H), 2.39 (s, 3H) |
| 1-2 | 2-Methyl-*N-*(2-phenyl-3*H-*imidazo[4,5-*b*]pyridin-6-yl)-5-((E)-3-pyridin-3-yl-acryloylamino)-benzamide | (400 MHz, D6-DMSO) 13.50 (br s) and 13.09 (br s, together 1H), 10.61 (br s) and 10.29 (br s, together 1H), 10.41 (s, 1H), 8.83 (s, 1H), 8.70 - 8.41 (m, 3H), 8.21 (m, 2H), 8.04 (s, 1H), 7.88 (s, 1H), 7.61 (m, 6H), 7.31 (d, 1H), 6.94 (d, 1H), 2.39 (s, 3H) |
| 1-3 | 2-Methyl-5-(4-morpholin-4-yl-benzoylamino)-*N-*(2-phenyl-3*H-*imidazo[4,5-b]pyridin-6-yl)-benzamide | (400 MHz, D6-DMSO) 13.54 (br s) and 13.08 (br s, together 1H), 10.63 (s) and 10.52 (s, together 1H), 10.07 (s, 1H), 8.70 - 8.45 (m, 2H), 8.22 (m, 2H), 7.92 (m, 4H), 7.58 (m, 3H), 7.29 (d, 1H), 7.04 (d, 2H), 3.75 (m, 4H), 3.26 (m, 4H), 2.39 (s, 3H) |
| 1-4 | 2-Methyl-5-[(E)-(3-phenyl-acryloyl)amino]-*N-*(2-phenyl-3*H-*imidazo[4,5-*b*]pyridin-6-yl)-benzamide | (400 MHz, D6-DMSO) 13.56 (br s) and 13.09 (br s, together 1H); 10.66 (s) and 10.55 (s, together 1H); 10.34 (s, 1H); 8.63-8.58 (m) and 8.47 (s, together 2H); 8.25 (d, 1H), 8.20 (d, 1H); 7.88 (s, 1H); 7.75 (d, 1H); 7.68-7.52 (m, 6H), 7.48-7.42 (m, 3H); 7.31 (d, 1H), 6.85 (d, 1H); 2.39 (s, 3H). |
| 1-5 | 5-Benzoylamino-2-methyl-*N-*(2-phenyl-3*H-*imidazo[4,5-*b*]pyridin-6-yl)-benzamide | (400 MHz, D6-DMSO) 13.55 (br s) and 13.09 (br s, together 1H); 10.66 (s) and 10.54 (s, together 1H); 10.37 (s, 1H); 8.63-8.58 (m) and 8.47 (s, together 2H); 8.24 (d, 1H); 8.19 (d, 1H); 8.00-7.93 (m, 3H), 7.86 (d, 1H); 7.65-7.50 (m, 6H); 7.33 (d, 1H); 2.40 (s, 3H). |
| 1-6 | Biphenyl-3-carboxylic acid [4-methyl-3-(2-phenyl-3*H-*imidazo[4,5-b]pyridin-6-ylcarbamoyl)-phenyl] -amide | (400 MHz, D6-DMSO) 13.58 (br s) and 13.10 (br s, together 1H); 10.67 (s) and 10.56 (s, together 1H); 10.46 (s, 1H); 8.63-8.58 (m) and 8.48 (s, together 2H); 8.26-8.18 (m, 3H); 7.98-7.89 (m, 4H), 7.79 (d, 2H); 7.67-7.50 (m, 6H); 7.43 (t, 1H); 7.35 (d, 1H); 2.41 (s, 3H). |
| 1-7 | Biphenyl-4-carboxylic acid [4-methyl-3-(2-phenyl-3*H*-imidazo[4,5-b]pyridin-6-ylcarbamoyl)-phenyl]-amide | (400 MHz, D6-DMSO) 13.54 (br s) and 13.11 (br s, together 1H), 10.61 (br s, 1H), 10.42 (s, 1H), 8.62 (s, 1H), 8.54 (br s, 1H), 8.22 (d, 2H), 8.10 (d, 2H), 8.01 (d, 1H), 7.87 (m, 3H), 7.77 (d, 2H), 7.55 (m, 5H), 7.43(m, 1H), 7.34 (d, 1H), 2.41 (s, 3H) |

### Examples 2-1:

### 2-Chloro-5-(4-morpholin-4-yl-benzoylamino)-N-(2-phenyl-3H-imidazo[4,5-b]pyridin-6-yl)-benzamide

Analogous to Example 1-1 from 31.5 mg 5-Amino-2-chloro-N-(2-phenyl-3H-imidazo[4,5-b]pyridin-6-yl)-benzamide from Example d) and 43 mg 4-morpholinobenzoyl chloride.
Yield 37 mg.

Using the experimental conditions reported above (Example 2-1) and the appropriate starting materials, the following derivatives were prepared:

| **Example No.** | **Systematic name** | **¹H-NMR** |
|---|---|---|
| 2-1 | 2-Chloro-5-(4-morpholin-4-yl-benzoylamino)-*N-*(2-phenyl-3*H-*imidazo[4,5-b]pyridin-6-yl)-benzamide | (400 MHz, D6-DMSO) 13.57 (br s) and 13.12 (br s, together 1H), 10.85 (br s) and 10.75 (br s, together 1H), 10.23 (s, 1H), 8.57 (m) and 8.45 (br s, 2H), 8.22 (m, 2H), 8.09 (s, 1H), 7.95 (m, 3H), 7.57 (m, 4H), 7.02 (m, 2H), 3.75 (m, 4H), 3.28 (m, 4H) |
| 2-2 | 2-Chloro-5-[(E)-(3-phenyl-acryloyl)amino]-*N-*(2-phenyl-3*H-*imidazo[4,5-b] pyridin-6-yl)-benzamide | (400 MHz, D6-DMSO) 13.58 (br s) and 13.13 (br s, together 1H); 10.87 (s) and 10.77 (s, together 1H); 10.54 (s, 1H); 8.61-8.56 (m) and 8.46 (s, together 2H); 8.22 (d, 2H); 8.01 (s, 1H); 7.84 (d, 1H); 7.67-7.57 (m, 7H), 7.47-7.44 (m, 3H); 6.84 (d, 1H). |
| 2-3 | Biphenyl-4-carboxylic acid [4-chloro-3-(2-phenyl-3*H-*imidazo[4,5-b]pyridin-6-ylcarbamoyl)-phenyl]-amide | (400 MHz, D6-DMSO) 12.95 (br s, 1H), 10.83 (br s, 1H), 10.59 (s, 1H), 8.59 (s) and 8.52 (br s, together 2H), 8.23 (d, 2H), 8.11 (m, 3H), 8.00 (d, 1H), 7.87 (d, 2H), 7.78 (d, 2H), 7.56 (m, 6H), 7.44 (m, 1H) |
| 2-4 | Biphenyl-3-carboxylic acid [4-chloro-3-(2-phenyl-3*H-*imidazo[4,5-b]pyridin-6-ylcarbamoyl)-phenyl] -amide | (400 MHz, D6-DMSO) 13.75 (br s) and 13.11 (br, s, together 1H), 10.88 (br s) and 10.77 (br s, together 1H), 10.61 (s, 1H), 8.53 - 8.50 (m) and 8.45 (br s, together 2H), 8.22 (m, 3H), 8.09 (d, 1H), 8.00 (d, 1H), 7.97 (d, 1H), 7.91 (d, 1H), 7.78 (d, 2H), 7.59 (m, 7H), 7.43 (m, 1H) |
| 2-5 | 5-Benzoylamino-2-chloro-*N-*(2-phenyl-3*H*-imidazo[4,5-b]pyridin-6-yl)-benzamide | (400 MHz, D6-DMSO) 13.19 (br s, 1H), 10.78 (br s, 1H), 10.54 (br s, 1H), 8.52 (d, 2H), 8.32 and 7.84 (m, 6H), 7.57 (m, 7H) |

### Example 3-1:

### 3 2-Methyl-5-[(E)-3-(3-nitro-phenyl)-acryloylamino]-N-(2-phenyl-3H-imidazo[4,5-b]pyridin-6-yl)-benzamide

115 mg 5-Amino-2-methyl-N-(2-pheny)-3H-imidazo[4,5-b]pyridin-6-yl)-benzamide from Example f) in 10 ml dry N-methyl-pyrrolidone (NMP) were cooled to 0 C. 71 mg (E)-3-nitro-cinnamoyl chloride were added and the mixture was stirred for 30 min at 0 C and another 2 hrs at room temperature. NMP was removed under vacuum and the residue was dissolved in 3 ml methanol and stirred with 1 ml conc. aqueous ammonia to cleave bis-acylated byproducts. After 1 hr the mixture was evaporated to dryness. The residue was dispersed in water. The crude product was isolated by filtration and washed thoroughly with water and subsequently with ether to give, after drying, 168 mg of the title product.

Using the experimental conditions reported above (Example 3-1) and the appropriate starting materials, the following derivatives were prepared:

| **Example No.** | **Systematic name** | **MS (M+H, method)** |
|---|---|---|
| 3-1 | 2-Methyl-5-[(E)-3-(3-nitro-phenyl)-acryloylamino]-N-(2-phenyl-3H-imidazo[4,5-b]pyridin-6-yl)-benzamide | M = 519.0 (API+) |
| 3-2 | 2-Methyl-5-[(E)-3-(2-nitro-phenyl)-acryloylamino]-N-(2-phenyl-3H-imidazo[4,5-b]pyridin-6-yl)-benzamide | M = 519.3 (ESI+) |
| 3-3 | 2-Methyl-5-[(E)-3-(4-nitro-phenyl)-acryloylamino]-N-(2-phenyl-3H-imidazo[4,5-b]pyridin-6-yl)-benzamide | M = 519.3 (ESI+) |

## Claims

1. A compound according to formula I, 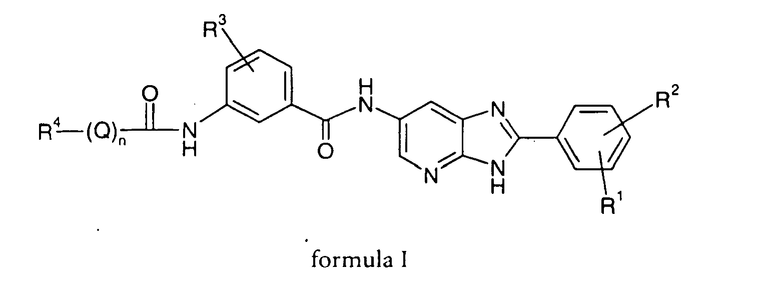 wherein,
R¹ and R² independently represent hydrogen, halogen, alkylamino, dialkylamino, alkoxy or alkyl, wherein the alkyl or the alkoxy are optionally substituted one or two times by alkoxy;
R³ is hydrogen, halogen, alkoxy or alkyl, wherein the alkyl or the alkoxy are optionally substituted one or several times by halogen;
Q is alkylene or alkenylene;
n is 0 or 1;
R⁴ is phenyl or pyridyl, said phenyl being optionally substituted once by phenyl, and all aromatic groups being optionally substituted one or several times by -CN, -CHO, -OH, -O-alkyl, -NO₂, -NH₂, -NH-alkyl, -alkylene-NH₂, alkyl, halogen or heterocyclyl;
and all pharmaceutically acceptable salts thereof.

2. The compounds according to claim 1, wherein
R¹ and R² are hydrogen;
R³ is halogen or alkyl; and
Q is alkenylene.

3. The compounds according to any one of claim 1 or 2, wherein
n is 1; and
R⁴ is pyridyl.

4. The compounds according to any one of claim 1 or 2, wherein
n is 0.
R⁴ is phenyl, said phenyl being optionally substituted once by phenyl or heterocyclyl.

5. The compounds according to claim 1, selected from the group consisting of:
2-Methyl-*N*-(2-phenyl-3*H*-imidazo[4,5*-b*]pyridin-6-yl)-5-((E)-3-pyridin-4-yl-acryloylamino)-ben-r.amide;
2-Methyl-*N-*(2-phenyl-3*H-*imidazo[4,5*-b*]pyridin-6-yl)-5-((E)-3-pyridin-3-yl-acryloylamino)-benzamide;
2-Methyl-5-(4-morpholin-4-yl-benzoylamino)-*N-*(2-phenyl-3*H-*imidazo[4,5*-b*]pyridin-6-yl)-benzamide;
2-Methyl-5- [(E)-(3-phenyl-acryloyl)amino] -*N-*(2-phenyl-3*H-*imidazo[4,5-b]pyridin-6-yl)-benzamide;
5-Benzoylamino-2-methyl-*N-*(2-phenyl-3*H-*imidazo [4,5*-b*]pyridin-6-yl)-benzamide;
Biphenyl-3-carboxylic acid [4-methyl-3-(2-phenyl-3*H-*imidazo[4,5-b]pyridin-6-ylcarbamoyl)-phenyl]-amide;
Biphenyl-4-carboxylic acid [4-methyl-3-(2-phenyl-3*H-*imidazo[4,5-b]pyridin-6-ylcarbamoyl)-phenyl]-amide;
2-Chloro-5-(4-morpholin-4-yl-benzoylamino)-*N-*(2-phenyl-3*H-*imidazo[4,5*-b*] pyridin-6-yl)-benzamide;
2-Chloro-5-[(E)-(3-phenyl-acryloyl)amino]-*N-*(2-phenyl-3*H-*imidazo[4,5-b] pyridin-6-yl)-benzamide;
Biphenyl-4-carboxylic acid [4-chloro-3-(2-phenyl-3*H-*imidazo[4,5-b]pyridin-6-ylcarbamoyl)-phenyl]-amide;
Biphenyl-3-carboxylic acid [4-chloro-3-(2-phenyl-3*H-*imidazo[4,5-b]pyridin-6-ylcarbamoyl)-phenyl]-amide;
5-Benzoylamino-2-chloro-*N-*(2-pheny)-3*H-*imidazo[4,5*-b*]pyridin-6-yl)-benzamide;
2-Methyl-5-[(E)-3-(3-nitro-phenyl)-acryloylamino]-N-(2-phenyl-3H-imidazo[4,5-b] pyridin-6-yl)-benzamide;
2-Methyl-5-[(E)-3-(2-nitro-phenyl)-acryloylamino]-N-(2-phenyl-3H-imidazo[4,5-b]pyridin-6-yl)-benzamide; and
2-Methyl-5-[(E)-3-(4-nitro-phcnyl)-acryloylamino]-N-(2-phenyl-3H-imidazo[4,5-b]pyridin-6-yl)-benzamide.

6. A process for the manufacture of the compounds of formula I according to claim 1, wherein
(a) the compound of formula IX, 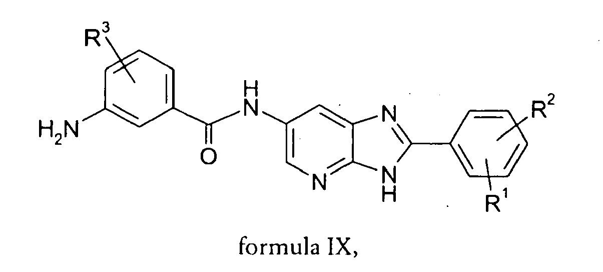 wherein R¹, R² and R³ have the significance as given above for formula I in claim 1,
is reacted with a compound of formula X, 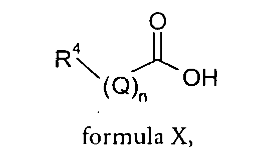 wherein R⁴, Q and n have the significance given above for formula I in claim 1, and wherein the carboxylic acid group is activated before the reaction,
to give the respective compound of formula I,
(b) said compound of formula I is isolated from the reaction mixture, and
(c) if desired, converted into a pharmaceutically acceptable salt.

7. A pharmaceutical composition, containing one or more compounds according to claims 1 to 5 as active ingredients together with pharmaceutically acceptable carriers.

8. The pharmaceutical composition according to claim 7 for the treatment of cancer.

9. Compounds of formula (I) according to claims 1-5 for use in a method of treating cancer.

## Patentansprüche

1. Verbindung nach Formel I, 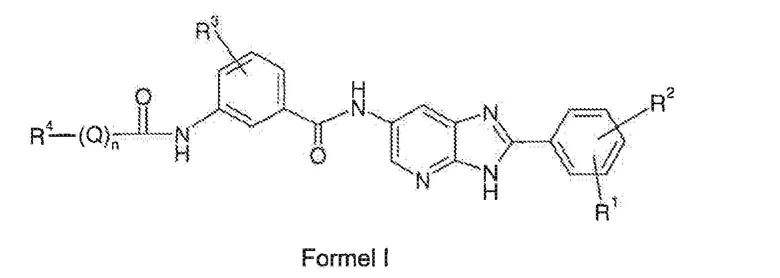 wobei
R¹ und R² unabhängig voneinander Wasserstoff, Halogen, Alkylamino, Dialkylamino, Alkoxy oder Alkyl darstellen, wobei das Alkyl oder das Alkoxy gegebenenfalls ein- oder zweimal mit Alkoxy substituiert sind;
R³ Wasserstoff, Halogen, Alkoxy oder Alkyl ist, wobei das Alkyl oder das Alkoxy gegebenenfalls einmal oder mehrmals mit Halogen substituiert sind;
Q Alkylen oder Alkenylen ist;
n 0 oder 1 ist;
R⁴ Phenyl oder Pyridyl ist, wobei das Phenyl gegebenenfalls einmal mit Phenyl substituiert ist, und alle aromatischen Gruppen gegebenenfalls einmal oder mehrmals mit -CN, -CHO, -OH, -O-Alkyl, -NO₂, -NH₂, -NH-Alkyl, -Alkylen-NH₂, Alkyl, Halogen oder Heterocyclyl substituiert sind;
und alle pharmazeutisch akzeptablen Salze davon.

2. Verbindungen nach Anspruch 1, wobei
R¹ und R² Wasserstoff sind,
R³ Halogen oder Alkyl ist; und
Q Alkenylen ist.

3. Verbindungen nach einem der Ansprüche 1 oder 2, wobei
n 1 ist; und
R⁴ Pyridyl ist.

4. Verbindungen nach einem der Ansprüche 1 oder 2, wobei
n 0 ist,
R⁴ Phenyl ist, wobei das Phenyl gegebenenfalls einmal mit Phenyl oder Heterocyclyl substituiert ist.

5. Verbindungen nach Anspruch 1, ausgewählt aus der Gruppe bestehend aus:
2-Methyl-*N*-(2-phenyl-3*H*-Imidazo[4,5-*b*]pyridin-6-yl)-5-((E)-3-pyridin-4-yl-acryloylamino)-benzamid;
2-Methyl-*N*-(2-phenyl-3*H*-imidazo[4,5*-b*]pyridin-6-yl)-5-((E)-3-pyridin-3-yl-acryloylamino)-benzamid;
2-Methyl-5-(4-morpholin-4-yl-benzoylamino)-*N*-(2-phenyl-3*H-*imidazo[4,5*-b*]pyridin-6-yl)-benzamid;
2-Methyl-5-[(E)-(3-phenyl-acryloyl)amino]-*N*-(2-phenyl-3*H-*imidazo[4,5*-b*]pyridin-6-yl)-benzamid;
5-Benzoylamino-2-methyl-*N*-(2-phenyl-3*H*-imidazo[4,5-b]pyridin-6-yl)-benzamid;
Biphenyl-3-carbonsäure [4-methyl-3-(2-phenyl-3*H*-imidazo[4,5-b]pyridin-6-ylcarbamoyl)-phenyl]-amid;
Biphenyl-4-carbonsäure [4-methyl-3-(2-phenyl-3*H*-imidazo[4,5-b]pyridin-6-ylcarbamoyl)-phenyl]-amid;
2-Chlor-5-(4-morpholin-4-yl-benzoylamino)-*N*-(2-phenyl-3*H-*imidazo [4,5*-b*]pyridin-6-yl)-benzamid;
2-Chlor-5-[(E)-(3-phenyl-acryloyl)amino]-*N*-(2-phenyl-3*H-*imidazo[4,5*-b*]pyridin-6-yl)-benzamid;
Biphenyl-4-carbonsäure [4-chlor-3-(2-phenyl-3*H*-imidazo[4,5-b]pyridin-6-ylcarbamoyl)-phenyl]-amid;
Biphenyl-3-carbonsäure [4-chlor-3-(2-phenyl-3*H*-imidazo[4,5-b]pyridin-6-ylcarbamoyl)-phenyl]-amid;
5-Benzoylamino-2-chlor-*N*-(2-phenyl-3*H*-imidazo[4,5-b]pyridin-6-yl)-benzamid;
2-Methyl-5-[(E)-3-(3-nitro-phenyl)-acryloylamino]-*N*-(2-phenyl-3*H-*imidazo[4,5*-b*]pyridin-6-yl)-benzamidi
2-Methyl-5-[(E)-3-(2-nitro-phenyl)-acryloylamino]-*N*-(2-phenyl-3*H-*imidazo[4,5*-b*]pyridin-6-yl)-benzamid-, und
2-Methyl-5-[(E)-3-(4-nitro-phenyl)-acryloylamino]-*N*-(2-phenyl-3*H-*imidazo[4,5-b]pyridin-6-yl)-benzamid.

6. Verfahren zur Herstellung der Verbindungen der Formel nach Anspruch 1, wobei
(a) die Verbindung der Formel IX, 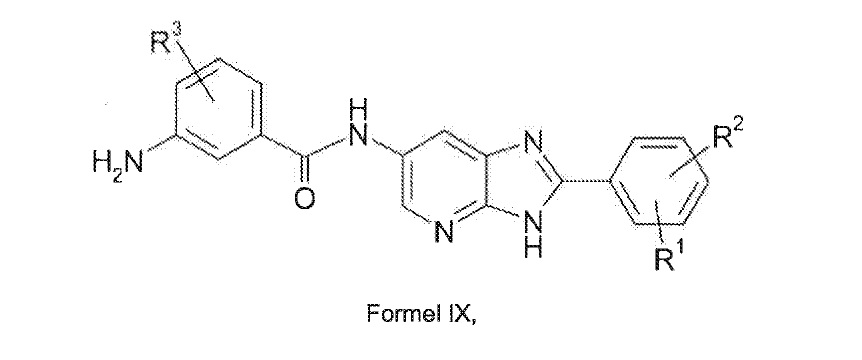 wobei R¹, R² und R³ die Bedeutung wie oben für Formel I in Anspruch 1 gegeben haben, mit einer Verbindung der Formel X umgesetzt wird, 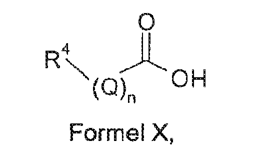 wobei R⁴, Q und n die Bedeutung wie oben für Formel I in Anspruch 1 gegeben haben, und wobei die Carbonsäuregruppe vor der Reaktion aktiviert wird,
um die entsprechende Verbindung der Formel I zu ergeben,
(b) die Verbindung der Formel I aus der Reaktionsmischung isoliert wird, und
(c) wenn benötigt, in ein pharmazeutisch akzeptables Salz umgewandelt wird.

7. Pharmazeutische Zusammensetzung, die eine oder meherere Verbindungen nach Ansprüchen 1 bis 5 als Wirkstoffe zusammen mit pharmazeutisch akzeptablen Trägern enthält.

8. Pharmazeutische Zusammensetzung nach Anspruch 7 für die Behandlung von Krebs.

9. Verbindungen der Formel (I) nach Ansprüchen 1 bis 5 für die Verwendung in einem Verfahren zur Behandlung von Krebs.

## Revendications

1. Composé selon la formule I 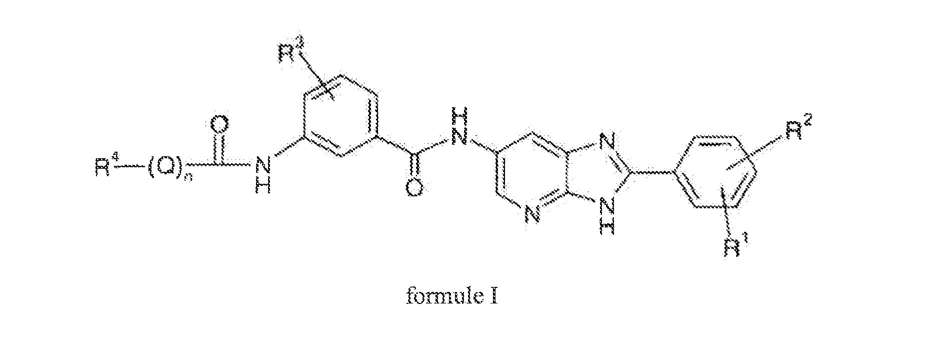 dans laquelle
R¹ et R² représentent indépendamment un atome d'hydrogène, d'halogène, un groupe alkylammo, dialkylamino, alcoxy ou alkyle, les groupes alkyl ou alkoxy étant éventuellement substitués une à deux fois par un groupe alkoxy ;
R³ est un atome d'hydrogène, d'halogène, un groupe alcoxy ou alkyle, les groupes alkyle ou alcoxy étant éventuellement substitués une ou plusieurs fois par un atome d'halogène ;
Q est un groupe alkylène ou alcényléne ;
n vaut 0 ou 1 ;
R⁴ est un groupe phényle ou pyridyle, ledit groupe phényle étant éventuellement substitué une fois par un groupe phényle et tous les groupes aromatiques étant éventuellement substitués une ou plusieurs fois par les groupes -CN, -CHO, -OH, -O-alkyl, -NO₂, -NH₂, -NH-alkyle, -alkylène-NH₂, alkyle, un atome d'halogène ou un groupe hétérocyclyle;
et tous leurs sels pharmaceutiquement acceptables.

2. Composés selon la revendication 1, dans lesquels
R¹ et R² sont un atome d'hydrogène ;
R³ est un atome d'halogène ou un groupe alkyle; et
Q est un groupe alcénylène.

3. Composés selon l'une quelconque des revendications 1 ou 2, dans lesquels
vaut 1 ; et
R⁴ est un groupe pyridyle.

4. Composés selon l'une quelconque des revendications 1 ou 2, dans lesquels
n vaut 0,
R⁴ est un groupe phényle, ledit groupe phényle étant éventuellement substitué une fois par un groupe phényle ou hétérocyclyle.

5. Composés selon la revendication 1, choisis dans le groupe comprenant :
le 2-méthyl-*N*-(2-phényl-3*H*-imidazo[4,5*-b*]pyridin-6-yl)-5-((E)-3-pyridin-4-yl-acryloylamino)-benzamide ;
le 2-méthyl-*N*-(2-phényl-3*H*-imidazo[4,5*-b*]pyridin-6-yl)-5-((E)-3-pyridin-3-yl-acryloylamino)-benzamide;
le 2-méthyl-5-(4-morpholin-4-yl-benzoylamino)-*N*-(2-phényl-3*H*-imidazo[4,5-*b*]pyridin-6-yl)-benzamide;
le 2-méthyl-5-[(E)-(3-phényl-acryloyl)amino]-*N*-(2-phényl-3*H*-imidazo[4,5-*b*]pyridin-6-yl)-benzamide;
le 5-benzoylamino-2-méthyl-*N*-(2-phényl-3*H*-imidazo[4,5*-b*]pyridin-6-yl)-benzamide ;
le [4-méthyl-3-(2-phényl-3*H*-imidazo[4,5*-b*]pyridin-6-ylcarbamoyl)-phényl]-amide de l'acide biphényl-3-carboxylique;
le [4-méthyl-3-(2-phényl-3*H*-imidazo[4,5*-b*]pyridin-6-ylcarbamoyl)-phényl]-amide de l'acide biphényl-3-carboxylique;
le 2-chloro-5-(4-morpholin-4-yl-benzoylamino)-*N*-(2-phényl-3*H*-imidazo[4,5-*b*]pyridin-6-yl)-benzamide;
le 2-chloro-5-[(E)-(3-phényl-acryloyl)amino]-*N*-(2-phényl-3*H*-imidazo[4,5-*b*]pyridin-6- yl)-benzamide ;
le [4-chloro-3-(2-phényl-3*H*-imidazo[4,5-b]pyridin-6-ylcarbamoyl)-phényl]-amide de l'acide biphényl-4-carboxylique;
le [4-chloro-3-(2-phényl-3*H*-imidazo[4,5-b]pyridin-6-ylcarbamoyl)-phényl]-amide de l'acide biphényl-3-carboxylique;
le 5-henzoylamino-2-chloro-*N*-(2-phényl-3*H*-imidazo[4,5*-b*]pyridin-6-yl)-benzamide ;
le 2-méthyl-5-[(E)-3-(3-nitro-phényl)-acryloylamino]-*N*-(2-phenyl-3*H*-imidazo[4,5-*b*]pyridin-6-yl)-benzamide ;
le 2-méthyl-5-[(E)-3-(2-nitro-phényl)-acryloylamino]-*N-*(2-phényl-3*H*-imidazo[4,5-*b*]pyridin-6-yl)-benzamide ; et
le 2-méthyl-5-[(E)-3-(4-nitro-phényl)-acryloylamino]-*N*-(2-phényl-3*H-*imidazo[4,5-*b*]pyridin-6-yl)-benzamide.

6. Procédé de production des composés de formule selon la revendication 1, dans lequel
(a) on fait réagir un composé de formule IX, 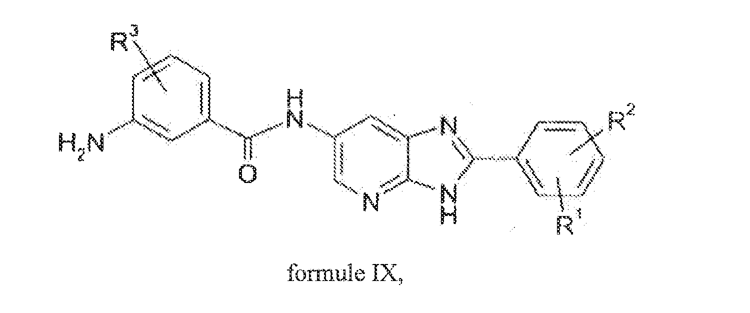 dans laquelle R¹, R² et R³ ont la signification indiquée ci-dessus pour la formule I de la revendication 1,
avec un composé de formule X, 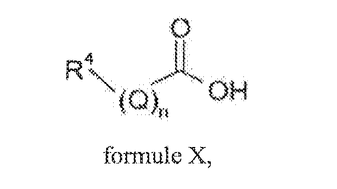 dans laquelle R⁴, Q et n ont la signification indiquée ci-dessus pour la formule I de la revendication 1, et dans laquelle on active le groupe acide carboxylique avant la réaction,
de manière à obtenir le composé correspondant de formule I,
(b) ledit composé de formule I est isolé du mélange réactionnel, et
(c) si on le souhaite,est converti en un sel pharmaceutiquement acceptable.

7. Composition pharmaceutique contenant un ou plusieurs composés selon les revendications 1 à 5, comme ingrédients actifs, conjointement avec des véhicules pharmaceutiquement acceptables.

8. Composition pharmaceutique selon la revendication 7, pour le traitement du cancer,

9. Composés de formule (I) selon les revendications 1 à 5, à utiliser dans un procédé de traitement du cancer.
